(19) Europäisches Patentamt
European Patent Office
Office européen des brevets

(11) Veröffentlichungsnummer: **0 183 150**
**B1**

# (12) EUROPÄISCHE PATENTSCHRIFT

(45) Veröffentlichungstag der Patentschrift: **18.04.90**

(51) Int. Cl.[5]: **C 08 G 18/77**, C 08 G 18/80, C 09 D 175/04

(21) Anmeldenummer: **85114567.2**

(22) Anmeldetag: **16.11.85**

## (54) Verfahren zur Herstellung von modifizierten Polyisocyanaten und ihre Verwendung in Polyurethananlacken.

(30) Priorität: **28.11.84 DE 3443342**

(43) Veröffentlichungstag der Anmeldung:
**04.06.86 Patentblatt 86/23**

(45) Bekanntmachung des Hinweises auf die Patenterteilung:
**18.04.90 Patentblatt 90/16**

(84) Benannte Vertragsstaaten:
**BE DE FR GB IT NL**

(56) Entgegenhaltungen:
**EP-A-0 054 491**
**EP-A-0 056 167**
**US-A-4 163 094**

(73) Patentinhaber: **BAYER AG**
**D-5090 Leverkusen 1 Bayerwerk (DE)**

(72) Erfinder: **König, Klaus, Dr.**
**Heymann-Strasse 50**
**D-5090 Leverkusen (DE)**
Erfinder: **Pedain, Josef, Dr.**
**Haferkamp 6**
**D-5000 Koeln 80 (DE)**
Erfinder: **Woynar, Helmut, Dr.**
**Ahornweg 7**
**D-4047 Dormagen (DE)**

EP 0 183 150 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft ein Verfahren zur Herstellung von neuartigen, modifizierten, zugleich Urethan-, Carbonsäureanhydrid- und Biuretgruppen aufweisenden Polyisocyanaten durch Umsetzung von einfachen Ausgangsdiisocyanaten mit bestimmten Hydroxycarbonsäuren und gegebenenfalls Wesser und ihre Verwendung, gegebenenfalls in mit Blockierungsmitteln für Isocyanatgruppen blockierter Form, also Polyisocyanatkomponente in Polyurethanlacken.

Modifizierte aliphatische Diisocyanate, insbesondere auf der Basis von Hexamethylendiisocyanat, haben weltweit für die Herstellung lichtechter und extrem witterungsbeständiger Lacke mit höchster Glanzhaltung technische Bedeutung erlangt. Für den Einsatz auf diesem Gebiet, insbesondere für klare und weiß-pigmentierte Beschichtungen, werden vom Markt wenig bis nicht gefärbte Produkte verlangt. Weiterhin ist für eine gefahrlose Verarbeitung ein möglichst geringer Anteil an monomeren Diisocyanaten anzustreben, der sich auch bei längerer Lagerung nicht erhöht. Augrund toxikologischer Untersuchungen ist eine gefahrlose Verarbeitung bis zu einem maximalen Gehalt von 0,7% en monomerem Diisocyanat gegeben, sofern die bei der Lackverarbeitung üblichen Schutzvorschiften eingehalten werden. Der vorgenannte Grenzwert hat in die Literatur (z.B. Merkblatt "PUR-Anstrichstoffe" des Hauptverbandes der deutschen gewerblichen Berufsgenossenschaft sowie "Polyurethane Report" der Paintmakers Ass.) Eingang gefunden.

Für die Herstellung solcher Polyisocyanate sind im Laufe der Zeite eine Vielzahl von unterschiedlichen Modifizierungsarten bekannt geworden. So haben sich beispielsweise bislang die folgenden Modifizierungsprodukte aliphatischer Diisocyanate in der Praxis bewährt:

—Uretdiongruppen enthaltende Polyisocyanate, hergestellt durch partielle Dimerisierung von Diisocyanaten in Gegenwart spezieller Katalysatoren

—Isocyanuratgruppen enthaltende Polyisocyanate, hergestellt durch partielle Trimerisierung von Diisocyanaten in Gegenwart spezieller Katalysatoren

—Polyisocyanatgemisch, die sowohl Isocyanurat- als auch Uretdiongruppierungen aufweisen, hergestellt durch partielle Trimerisierung ind gleichzeitige partielle Dimerisierung von Diisocyanaten in Gegenwart spezieller Katalysatoren, die sowohl die Trimerisierung als auch die Dimerisierung von Isocyanatgruppen beschleunigen

—Urethangruppe enthaltende Polyisocyanate durch partielle Umsetzung von Diisocyanaten mit Polyolen, üblicherweise mit Triolen

—Biuretgruppen enthaltende Polyisocyanate durch partielle Umsetzung von Diisocyanaten mit einem geeigneten Biuretisierungsmittel.

Diese, an sich in der Praxis bewährten, modifizierten Lackpolyisocyanate weisen jedoch nach wie vor verbesserungswürdige Nachteile auf.

Die Uretdion- und/oder Isocyanuratgruppen aufweisenden Polyisocyanate werden im allgemeinen unter Verwendung spezieller Katalysatoren hergestellt (z.B. phosphorhaltige Substanzen, tert. Amine oder Alkalimetall-Carboxylate), die zwecks Abbruch der Modifizierungsreaktion beim angestrebten Modifizierungsgrad im allgemeinen durch Zugabe eines Abstoppers vernichtet werden, wobei die Folgeprodukte (Umsetzungsprodukte des Katalysators mit dem Abstopper) in den meisten Fällen nicht vom Endprodukt abgetrennt werden können und dort beispielsweise Trübungen oder nachteilhafte Produkteigenschaften bei der späteren Verwendung bewirken können.

Zwar können auch Katalysatoren eingesetzt werden, die mit fortschreitender Reaktion und bei höheren Temperaturen ihre Wirksamkeit verlieren. Bei Einsatz solcher Verbindungen werden zwar keine zusätzlichen Abstopper benötigt, jedoch ist die genaue Dosierung der Katalysatoren zur Erreichung exakt definierter Umsätze an Isocyanatgruppen problematisch. Auch in diesem Fall verbleiben die Zersetzungsprodukte der Katalysatoren im Produkt mit den bereits erwähnten möglichen Nachteilen.

Bei der Herstellung von Urethangruppen aufweisenden Polyisocyanaten entfällt zwar die Notwendigkeit der Mitverwendung spezieller Katalysatoren, jedoch weisen die bekannten, ausschließlich Urethangruppen enthaltenden, modifizierten Polyisocyanate oftmals extrem hohe Viskositäten auf, die ihre Verwendung, beispielsweise zur Herstellung von Beschichtungen, stark einschränken, da zum Einstellen verarbeitungsgerechter Viskositäten stark mit Lösungsmitteln verdünnt werden muß. Gerade in neuerer Zeit besteht jedoch ein deutlicher Trend zu lösungsmittelarmen oder sogar zu lösungsmittelfreien Systemen, bedingt durch sowohl arbeitshygienische und ökologische als auch ökonomische Forderungen.

Die Biuretgruppen enthaltenden Lackpolyisocyanate des Standes der Technik können nach einer Vielzahl unterschiedlicher Verfahren hergestellt werden, wobei es auch möglich ist, die Viskosität der modifizierten Polyisocyanate dem jeweiligen Einsatzzweck anzupassen. Die zur Herstellung der Biuretgruppen aufweisenden Polyisocyanate eingesetzten Modifizierungsmittel (Biuretisierungsmittel wie z.B. Wasser, wasserabspaltende Verbindungen oder Amine der unterschliedlichsten Art) sind jedoch mit speziellen Nachteilen behaftet, die sich entweder auf die Herstellungsweise oder die Produkteigenschaften in nachteiliger Weise auswirken.

Benutzt man beispielsweise Wasser als Biuretisierungsmittel, so benötigt man große Mengen eines organischen Lösungsmittels, um die normalerweise nicht mischbaren Komponenten Wasser und Diisocyanat in ein homogenes, zur Durchführung der Biuretisierung geeignetes Gemisch zu überführen. Trotz der Mitverwendung dieser Lösungsmittel kommt es hierbei oft zur Bildung von unlöslichen Polyharnstoffen, die nicht mehr als Biuret in Lösung gebracht werden

können. Neben der Notwendigkeit, große Mengen an Lösungsmitteln destillativ zu entfernen, ist daher oft noch ein zusätzlicher Filtrationsschritt notwendig.

Verfahren mit wasserabspaltenden Substanzen (z.B. tert. Butanol) führen einerseits zum Verlust des Biuretisierungsmittels, im angesprochenen Falls als Isobuten, das aufwendig entsorgt werden muß. Andererseits müssen bei diesen Verfahren Temperaturen von über 160°C über einen längeren Zeitraum aufrechterhalten werden, um eine vollständige Umsetzung zu erreichen. Diese Temperaturbelastung führt aber infolge von verstärkt auftretenden Nebenreaktionen zu schlechten Farbqualitäten der Endprodukte.

Die trifft auch dann zu, wenn Amine nicht in situ aus Isocyanaten erzeugt sondern das überschüssige Diisocyanat direkt mit dem entsprechenden Diamin zur Reaktion gebracht wird. Infolge der hohen Reaktivität der Amine gegenüber Isocyanaten kommt es dabei jedoch unweigerlich zur Bildung von hochmolekularen Polyharnstoffen, zu deren Auflösung unter Biuretbildung eine starke Temperaturbelastung notwendig ist, verbunden mit einer Verschlechterung der Farbqualität sowie dem vermehrten Auftreten von Nebenprodukten. Eine hohe Temperaturbelastung läßt sich auch dann nicht vermeiden, wenn spezielle Amine, die mit dem Diisocyanat keine hochschmelzenden Harnstoffe bilden, zur Anwendung kommen, oder wenn bei Verwendung von dampfförmigen Aminen die Bildung fester Harnstoffe unterbleibt, da sich an diese Umsetzungen notwendigerweise Equlibrierungsreaktionen, die nur bei hoher Temperatur ablaufen, anschließen müssen. Neben Uretdionen und Isocyanuraten treten dabei auch Carbodiimide bzw. Folgeprodukte von Carbodiimiden auf, die die Monomerenstabilität des Endproduktes ungünstig beeinflussen.

Es war daher die der Erfindungs zugrundeliegende Aufgabe, neue modifizierte Polyisocyanate zur Verfügung zu stellen, die allen an hochwertige Lackpolyisocyanate zu stellenden Anforderungen genügen, d.h. die insbesondere eine ausgezeichnete Farbqualität und gute Monomerenstabilität aufweisen, und die zudem in einfacher Weise und unter schonenden Bedingungen herstellbar sind.

Diese Aufgabe konnte mit der Bereitstellung des nachstehend näher beschriebenen erfindungsgemäßen Verfahrens gelöst werden. Bei diesem Verfahren werden bestimmte Ausgangsdiisocyanate mit ausgewählten Hydroxyalkancarbonsäuren bzw. deren Kombination mit Wasser unter Bildung von Urethan-, Carbonsäureanhydrid- und Biuretgruppen zur Umsetzung gebracht.

Die Herstellung von modifizierten Polyisocyanaten durch Umsetzung von einfachen Diisocyanaten mit wäßrigen Lösungen von wasserlöslichen Carbonsäuren ist zwar bereits aus der DE—OS—3 228 721 bekannt, jedoch führt die Umsetzung der Ausgangsdiisocyanate mit wäßrigen Lösungen der dort genannten Säuren zu unerwünschten Nebenprodukten, die über die Zwischenstufe von Carbonsäureamidgruppen gebildete Acylharnstoffgruppen aufweisen, was nicht nur zur Farbvertiefung der modifizierten Polyisocyanate gemäß Vorveröffentlichung führt, sondern was darüber hinaus den gravierenden Nachteil mit sich bringt, daß die modifizierten Polyisocyanate des genannten Standes der Technik bzw. die in ihnen enthaltenen Nebenprodukte mit Acylharnstoffgruppierungen instabil sind und zur Abspaltung der ihnen zugrundeliegenden monomeren Diisocyanate neigen. Dieser Nachteil tritt bei Verwendung der erfindungswesentlichen, ausgewählten Modifizierungsmittel nicht ein, vielmehr ermöglicht das nachstehend näher beschriebene erfindungsgemäße Verfahren die Herstellung von neuartigen, praktisch farblosen, monomerenstabilen Lackpolyisocyanaten.

Gemäß EP—A—00 56 167 werden organische Isocyanate ebenfalls mit Hydroxycarbonsäuren umgesetzt, jedoch handelt es sich bei den Hydroxycarbonsäuren nicht um solche, die in 2-Stellung zur Carboxylgruppe trisubstituiert sind, wie sie erfindungsgemäß eingesetzt werden. Die Reaktion zwischen Isocyanaten und Hydroxycarbonsäuren gemäß dieser Vorveröffentlichung dient auch einem völlig anderen Zweck, nämlich der Herstellung von Carbonxylgruppen aufweisenden Polyurethan-Prepolymeren.

Auch die US—PS—4 163 094 erwähnt die Umsetzung von organischen Isocyanaten mit Hydroxycarbonsäuren, wobei hier ebenfalls eine Säure (Dimethylolpropionsäure) verwendet wird, die auch erfindungsgemäß geeignet ist. Als Isocyanate werden teilblockierte Polyisocyanate eingesetzt, die jedoch nicht mit der reinen Hydroxycarbonsäure sondern mit einem Gemisch dieser Säure mit anderen Polyhydroxylverbindungen zu einem Carboxylgruppen aufweisenden Polyurethan umgesetzt werden. Wegen der hohen Konzentration an freien Hydroxylgruppen im Reaktionsgemisch reagieren diese bevorzugt mit den Isocyanatgruppen unter Urethanbildung ab, so daß die vergleichsweise reaktionsträgeren Carboxylgruppen nicht abreagieren und in das Polyurethangerüst eingebaut werden.

Gegenstand der Erfindung ist ein Verfahren zur Herstellung von modifizierten, zugleich Urethan-, Carbonsäureanhydrid- und Biuretgruppen aufweisenden organischen Polyisocyanaten durch Umsetzung von überschüssigen Mengen an organischen Diisocyanaten mit aliphatisch gebundenen Isocyanatgruppen des Molekulargewichtsbereichs 140—300 mit einem Modifizierungsmittel innerhalb des Temperaturbereichs von 20 bis 160°C, gegebenenfalls in Anwesenheit von mit Wasser mischbaren Lösungsmitteln, und gegebenenfalls unter anschließender Entfernung von überschüssigem, nicht umgesetztem Ausgangsdiisocyanat und sonstigen, gegebenenfalls vorliegenden flüchtigen Bestandteilen aus dem Reaktionsgemisch durch Destillation und/oder Extraktion, dadurch gekennzeichnet, daß man als Modifizierungsmittel

a) Hydroxycarbonsäuren der allgemeinen Formel

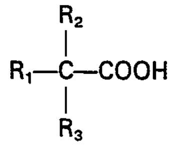

in welcher

$R_1$ für einen gegebenenfalls verzweigten, eine primäre oder sekundäre Hydroxylgruppe aufweisenden Alkylrest mit 1 bis 8 Kohlenstoffatomen,

$R_2$ einen gegebenenfalls verzweigten, gegebenenfalls eine primäre oder sekundäre Hydroxylgruppe aufweisenden Alkylrest mit 1 bis 8 Kohlenstoffatomen und

$R_3$ einen gegebenenfalls verzweigten, unsubstituierten Alkylrest mit 1 bis 14 Kohlenstoffatomen

bedeuten, wobei die Summe der Kohlenstoffatome der Reste $R^1$, $R^2$ und $R^3$ 3 bis 20 beträgt und, gegebenenfalls,

b) Wasser

verwendet, wobei ein Molverhältnis von a:b von 0,02:1 bis 1:0 eingehalten wird, und wobei die Menge des Modifizierungsmittels a) oder die Gesamtmenge der Modifizierungsmittelkomponenten a) und b) so bemessen werden, daß das Verhältnis (Mole Ausgangsdiisocyanat):(Gesamtmenge der Mole an Modifizierungsmittelkomponenten a) und b)) bei 3:1 bis 24:1 liegt.

Gegenstand der Erfindung ist auch die Verwendung der neuen Polyisocyanate, gegebenenfalls in mit Blockierungsmitteln für Isocyanatgruppen blockierter Form, als Polyisocyanatkomponente in Polyurethanlack-Systemen.

Für das erfindungsgemäße Verfahren geeignete Ausgangsdiisocyanate sind beliebige Diisocyanate mit aliphatisch gebundenen Isocyanatgruppen des Molekulargewichtsbereichs 140 bis 300. Bei den die Isocyanatgruppen dieser Diisocyanate verknüpfenden inerten Reste handelt es sich vorzugsweise um aliphatische Kohlenwasserstoffreste, die gegebenenfalls Estergruppen als Substituenten oder auch in der Hauptkette aufweisen können. Genannt seien beispielsweise: 1,4-Diisocyanatobutan, 1,5-Diisocyanatopentan, 1,6-Diisocyanatohexan, 1,8-Diisocyanatooctan, 1,10-Diisocyanatodecan, Isomerengemische aus 2,2,4-Trimethyl- und 2,4,4-Trimethyl-1,6-diisocyanatohexan, 2-Methyl-1,5-diisocyanato-pentan, 2,2-Dimethyldiisocyanato-pentan, 6-Isocyanatohexansäure-(2-isocyanatoethyl)-ester, 2,6-Diisocyanato-hexansäure-methylester. Grundsätzlich ist es auch möglich, die beispielhaft genannten aliphatischen Diisocyanate im Gemisch mit cycloaliphatischen Diisocyanaten wie z.B. 4,4'-Diisocyanato-dicyclohexylmethan oder Isophoron-diisocyanat einzusetzen, obwohl dies weniger bevorzugt ist. Ganz besonders bevorzugt wird 1,6-Diisocyanatohexan als alleiniges Ausgangsdiisocyanat beim erfindungsgemäßen Verfahren verwendet.

Unter "Modifizierungsmittel" sind im Rahmen der Erfindung gegenüber Isocyanatgruppen reaktionsfähige Verbindungen zu verstehen, die mit den Ausgangsdiisocyanaten unter gleichzeitiger Bildung von Urethan-, Carbonsäureanhydrid- und Biuretgruppen reagieren. Bei den erfindungswesentlichen Modifizierungsmitteln handelt es sich a) um bestimmte Hydroxycarbonsäuren oder um Kombinationen derartiger Hydroxycarbonsäuren mit b) Wasser.

Die für das erfindungsgemäße Verfahren geeigneten Hydroxycarbonsäuren a) entsprechen der allgemeinen Formel

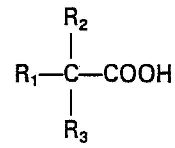

in welcher

$R_1$, $R_2$ und $R_3$ die bereits obengenannte Bedeutung haben.

Vorzugsweise werden solche Hydroxycarbonsäuren der genannten allgemeinen Formel eingesetzt, für welche

$R_1$ für einen Hydroxymethylrest,

$R_2$ für einen Hydroxymethyl-, Methyl- oder Ethylrest und

$R^3$ für einen gegebenenfalls verzweigten Alkylrest mit 1 bis 4 Kohlenstoffatomen stehen.

Typische Beispiele für derartige Hydroxycarbonsäuren sind 2-Hydromethyl-2-methyl-pentansäure, 2-Hydroxymethyl-2-ethyl-hexansäure, 3-Hydroxypivalinsäure und 2,2-Dimethylolpropionsäure. Die beiden letztgenannten Säuren werden bevorzugt verwendet, die 3-Hydroxypivalinsäure ist besonders bevorzugt.

Gemäß einer ersten Variante des erfindungsgemäßen Verfahrens können die beispielhaft genannten Hydroxycarbonsäuren als alleinige Modifizierungsmittel eingesetzt werden. Gemäß einer zweiten Variante des Verfahrens gelangen die erfindungsgemäßen Hydroxycarbonsäuren in Kombination mit Wasser zum Einsatz, so daß die Kombination aus Hydroxycarbonsäure und Wasser das Modifizierungsmittel darstellt. Im allgemeinen werden die Modifizierungsmittel a) und gegebenenfalls b) in einem Molverhältnis Hydroxycarbonsäure:Wasser von 0,02:1 bis 1:0 vorzugsweise von 0,05:1 bis 1:0 eingesetzt, wobei sich die molare Menge an Ausgangsdiisocyanat zur gesamten molaren Menge des Modifizierungsmittel wie 3:1 bis 24:1, vorzugsweise 5:1 bis 15:1 verhält.

Grundsätzlich können natürlich zusätzlich zu den erfindungsgemäß einzusetzenden Modifizierungsmitteln weitere Modifizierungsmittel der an sich bekannten Art mitverwendet werden, jedoch ist dies weniger bevorzugt.

Es kann zweckmäßig sein, insbesondere bei Mitverwendung von Wasser, das erfindungsgemäße Verfahren in Gegenwart eines mit Wasser zumindest in gewissen Grenzen mischbaren, gegen Isocyanat-, Säure- und Hydroxyl-Gruppen unter den angewandten Reaktionsbedingungen inerten Lösungsmittels durchzuführen. Als gegebenenfalls mitzuverwendende Lösungsmittel seien beispielhaft genannt: Ether, wie Di-isopropylether, Ethylenglykoldimethylether, Diethylenglykoldimethylether, 1,4-Dioxan, Tetrahydrofuran, 1,2-Dimethoxypropan; Ester, wie Butyrolacton, Ethylenglykolcarbonat, Propylenglykolcarbonat; Ether-Ester, wie Methoxyethylacetat, Ethoxyethylacetat, 1-Methoxypropyl-2-acetat, 2-Methoxypropyl-1-acetat, 1-Ethoxypropyl-2-acetat, 2-Ethoxypropyl-1-acetat; Ketone, wie Aceton, Methylethylketon; Nitrile, wie Acetonitril, Propionitril, Methoxypropionitril; Sulfone, wie Sulfolan, Dimethylsulfon, Diethylsulfon, Phosphorsäureester, wie Triethylphosphat, Trimethylphosphat oder Gemische derartiger Lösungsmittel.

Als weniger bevorzugte Lösungsmittel seien genannt: Tetramethylharnstoff, N-Methylpyrrolidon, Dimethylformamid, Dimethylacetamid.

Das Verfahren wird bei Temperaturen von 20—160°C, vorzugsweise bei 50—140°C durchgeführt. Üblicherweise wird das Verfahren bei Normaldruck durchgeführt. Insbesondere bei Mitverwendung von Wasser und/oder niedrigsiedenden Lösungsmitteln kann das Verfahren natürlich auch bei Drücken von 1 bis 30 bar, vorzugsweise 1 bis 5 bar durchgeführt werden.

Die Hydroxylgruppen der erfindungswesentlichen Modifizierungsmittel a) reagieren mit einem Teil der Isocyanatgruppen des Ausgangsdiisocyanats unter Bildung von Urethangruppen und offensichtlich unter Wasserabspaltung aus den Carbonsäuregruppen unter Bildung von Carbonsäureanhydridgruppen unter gleichzeitiger Biuretisierung eines weiteren Teils der Isocyanatgruppen des Ausgangsdiisocyanats, wobei der genaue Reaktionsmechanismus noch weitgehend ungeklärt ist. Überraschenderweise erfolgen diese Reaktionen unter äußerst milden Bedingungen, beispielsweise bei Temperaturen von etwa 80°C, während die direkte Reaktion von Diisocyanaten mit Wasser in Abwesenheit der erfindungsgwesentlichen Modifizierungsmittel a) bekanntlich erheblich drastischere Reaktionsbedingungen erfordert. Im Falle der Verwendung von erfindungswesentlichen Modifizierungsmitteln a) in Kombination mit der Modifizierungsmittelkomponente b), nämlich Wasser, entfalten die Modifizierungsmittelkomponenten a) eine katalytische Wirkung bezüglich der Biuretbildung durch Reaktion eines Teils der Isocyanatgruppen mit dem Wasser (unter gleichzeitiger Bildung von Urethan- und Carbonsäureanhydridgruppen durch Reaktion eines weiteren Teils der Isocyanatgruppen mit der Komponente a)), so daß auch die Diisocyanat-Wasser-Reaktion bei deutlich niedrigeren Temperaturen durchgeführt werden kann als bei Abwesenheit der Hydroxycarbonsäuren a). Wenn als Modifizierungsmittel Kombinationen aus den Einzelkomponenten a) und b) eingesetzt werden, die vorwiegend aus Wasser bestehen, entstehen naturgemäß Polyisocyanate, die in erster Linie Biuretgruppen aufweisen, und die nur noch untergeordnete Mengen an Urethan- und Carbonsäureanhydridgruppen aufweisen. Grundsätzlich kann gesagt werden, daß die jeweilige Konzentration an Urethan-, Carbonsäureanhydrid- und Biuretgruppen in den erfindungsgemäßen Verfahrensprodukten in erster Linie vom Verhältnis der jeweils zum Einsatz gelangenden Modifizierungsmittelkomponenten a) und b) abhängt.

Das erfindungsgemäße Verfahren wird beispielsweise wie folgt durchgeführt:

Das einzusetzende Diisocyanat wird in einem Rührreaktor, der gegebenenfalls mit einer Meßeinrichtung für das entstehende Kohlendioxid versehen ist, bei Raumteperatur oder erhöhter Temperatur vorgelegt.

Wird eine Hydroxycarbonsäure als alleiniges Modifizierungsmittel eingesetzt, wird diese, sofern sie bei Raumtemperatur flüssig ist, bei dieser Temperatur im Verlaufe von ca. 30 min. in das vorgelegte Diisocyanat eindosiert, wobei eine exotherme Reaktion mit beginnender $CO_2$-Entwicklung einsetzt, die durch Aufheizen auf 60—80° mit vertretbarer Geschwindigkeit weitergeführt wird. Der gegen Ende der Reaktion einsetzenden Verlangsamung begegnet man durch Erwärmen auf 100—120°. Der Zeitaufwand für die gesamte Reaktion beträgt im allgemeinen 1—12 h, vorzugsweise 2—8 h.

Bei Raumtemperatur feste Hydroxycarbonsäuren werden im allgemeinen in dieser Form dem Diisocyanat zudosiert, wobei das Diisocyanat vorteilhaft bei erhöhter Temperatur von 80—120° vorgelegt wird. Die Zugabe der Säure erfolgt in dem Maße, wie die Reaktion fortschreitet, erkennbar beispielsweise an der $CO_2$-Entwicklung.

Wird eine Hydroxycarbonsäure zusammen mit Wasser als Modifizierungsmittel eingesetzt, ist es in jedem Falle von Vorteil, das Diisocyanat bei erhöhter Temperatur vorzulegen. In diesem Falle ist insbesondere auch die Mitverwendung eines Lösungsmittels als Lösungsvermittler zwischen Wasser und Diisocyanat vorteilhaft. Andernfalls kann es zu schwerlöslichen Polyharnstoffausfällungen im Reaktionsgemisch kommen. Weiterhin kann durch entweichendes Kohlendioxid Wasser mitgerissen werden, das dann an den oberen Teilen des Reaktionsgefäßes kondensiert oder in die Abluft gelangt und somit der Reaktion entzogen wird. Durch Zugabe eines Lösungsmittels mit einem geeigneten Siedepunkt, das mit Wasser zumindest in gewissen Grenzen mischbar ist, können diese unkontrollierten Wasserverlüste vermieden werden. Dabei ist jedoch die notwendige Lösungsmittelmenge deutlich geringer als bei Verfahren, die Wasser als alleiniges Modifizierungsmittel verwenden.

Das gegebenenfalls mitzuverwendende Lösungsmittel kann ganz oder teilweise zusammen mit dem Diisocyanat vorgelegt werden oder ganz oder teilweise mit der Hydroxycarbonsäure bzw. dem gegebenenfalls mitzuverwendenden Wasser dem Diisocyanat zudosiert werden.

Falls als Modifizierungsmittel Kombinationen von Hydroxycarbonsäure und Wasser verwendet werden können die beiden Einzelkomponenten sowohl getrennt als auch in Form einer wäßrigen Lösung der Hydroxycarbonsäure zum Einsatz gelangen. Im Falle der Mitverwendung von Wasser und/oder Lösungsmitteln kann es zweckmäßig sein, zur Vermeidung von Verlusten an diesen Komponenten unter Überdruck zu arbeiten. Vorteilhaft wird dabei der maximale Druck während der Reaktion durch geeignete Maßnahmen, z.B. durch ein Druckhalteventil, auf 5 bar begrenzt, das bis zu diesem Druck die Benutzung üblicher technischer Apparate problemlos möglich ist. Die Reaktion kann selbstverständlich auch unter höheren Drücken, z.B. unter dem vollständigen Eigendruck des bei der Reaktion entstehenden Kohlendioxids durchgeführt werden, wobei je nach Art der Temperaturführung und Befüllungsgrad des Reaktors Drücke bis zu maximal 20 bar auftreten können. In solchen Fällen sind jedoch spezielle Hochdruckapparaturen erforderlich.

Das Verhältnis von NCO-Gruppen zu Säure und Wasser kann, wie bereits ausgeführt, in weiten Grenzen variiert werden. Es bestimmt die Oligomerenverteilung des resultierenden Polyisocyanats und damit maßgebliche Eigenschaften des Produktes, z.B. Isocyanatgehalt und Viskosität.

Des weiteren können durch Art und Menge der eingesetzten Hydroxycarbonsäuren unterschiedliche Gruppierungen in die modifizierten Polyisocyanate eingeführt werden, die eine Anpassung der Produkteigenschaften an die gewünschten Einsatzgebiete erlauben. Nach Beendigung der Reaktion wird im allgemeinen überschüssiges Diisocyanat destillativ aus dem Reaktionsgemisch entfernt. Wurde ein Lösungsmittel mitverwendet, kann dieses, sofern es niedriger siedet als das eingesetzte Diisocyanat, vor diesem durch Destillation abgetrennt werden. Vorteilhafter wird es jedoch zusammen mit diesem abgetrennt, wenn Diisocyanat und Lösungsmittel anschließend wiederverwendet werden sollen.

Grundsätzlich ist es auch möglich, jedoch weniger bevorzugt, das überschüssige Diisocyanat beispielsweise nach Entfernung des gegebenenfalls mitverwendeten Lösungsmittels durch Extraktion, beispielsweise mit n-Hexan, von dem gebildeten Polyisocyanat abzutrennen.

Selbstverständlich eignet sich das Verfahren in hervorragender Weise auch für eine kontinuierliche Durchführung. In diesem Fall wird beispielsweise in den ersten von 4—6 kaskadenförmige hitereinander geschalteten Rührreaktoren Diisocyanat und Hydroxycarbonsäure, gegebenenfalls zusammen mit Wasser und/oder einem Lösungsmittel separat oder als Gemisch so zudosiert, daß sich bis zum Verlassen des letzten Reaktors eine Verweilzeit von 2—8 Stunden ergibt. Dabei kann beispielsweise die Temperatur in den einzelnen Reaktoren gleichförmig 100—120°C oder in den einzelnen Reaktoren ansteigend 20—140°C, besser 40—120°C betragen. Je nach den Siedepunkten von Diisocyanat und gegebenenfalls mitverwendetem Lösungsmittel kann das Reaktionsgemisch entweder zuerst über eine kontinuierlich zu betreibende Destillationskolonne zur Abtrennung des Lösungsmittels geleitet und anschließend das modifizierte Polyisocyanat durch Dünnschicht-Destillation oder Extraktion von überschüssigem Diisocyanat befreit werden, oder man kann zunächst das Polyisocyanat durch gemeinsame Dünnschichtverdampfung von überschüssigem Diisocyanat und dem Lösungsmittel befreien und anschließend Diisocyanat und Lösungsmittel destillativ trennen. Vorteilhafterweise wird man jedoch das Lösungsmittel zusammen mit dem Diisocyanat abtrennen und das Destillat ohne weitere Aufarbeitung in den Prozess zurückführen. Im allgemeinen werden die erfindungsgemäßen Verfahrensprodukte bis auf einen Restgehalt von max, 0,7 Gew.-% von monomeren Ausgangsdiisocyanaten befreit.

Die nach dem erfindungsgemäßen Verfahren hergestellten erfindungsgemäßen Polyisocyanate zeichnen sich durch hohe Farbqualität sowie gute Lagerstabilität aus und sind weitgehend frei von Nebenprodukten.

Aus den in den Verfahrensprodukten vorliegenden Säureanhydridgruppen entstehen bei der erfindungsgemäßen Verwendung der Polyisocyanate in Kombination mit Polyhydroxylverbindungen freie Säuregruppen, die für die Haftung von aus diesen Produkten hergestellten Überzügen auf Metall vorteilhaft sein können. Eine weitere Möglichkeit der Ausnutzung dieser Anhydridgruppierungen besteht beispielsweise darin, nach Blockierung der Isocyanat-Gruppen des Polyisocyanates durch ein geeignetes Blockierungsmittel, beispielsweise Caprolactam, Phenol, Malonester oder Butanonoxim, durch Hydrolyse Säuregruppen zu erzeugen, die dann als ionisches Zentrum für die Herstellung wasserlöslicher oder wasserdispergierbarer PU-Systeme dienen können, Grundätzlich können die erfindungsgemäßen Verfahrensprodukte für alle Einsatzgebiete verwendet werden, für welche bislang die eingangs genannten Lackpolyisocyanate eingesetzt worden sind. Die erfindungsgemäßen Verfahrensprodukte eignen sich insbesondere als Polyisocyanatkomponente in Polyurethanlack-Systemen.

Die folgenden Biespiele dienen der weiteren Erläuterung der Erfindung. Alle Prozentangaben beziehen sich auf Gewichtsprozente.

**Beispiel 1**

In einem 6 1-Vierhalskolben mit Kontaktthermometer, Rührer und Rückflußkühler werden 4032 g (24 Mol) 1,6-Diisocyanatohexan bie 20°C vorgelegt und mit 472 g (4 Mol) fester Hydroxypivalinsäure (2-Hydroxymethyl-2,2-dimethyl-essigsäure) versetzt. Das Reaktionsgemisch wird anschließend im Verlauf von etwa 2 h in einem Maße, wie es die bald einsetzende Gastenwicklung zuläßt, bis auf einen Endwert von 120°C aufgeheizt. Nach Erreichen dieser Temperatur wird noch etwa 1 h bis zur Beendigung der Gasentwicklung gerührt. Insgesamt werden korr. 49,5 l $CO_2$ freigesetzt. Das so erhaltene rohe Polyisocyanat mit einem NCO-Gehalt von 34,5% wird durch zweimalige Dünnschichtdestillation auf einem Kurzwegverdampfer der Firma Leybold-Heraeus, Modell KDT 6, von überschüssigem 1,6-Diisocyanatohexan befreit. Im ersten Schritt wird dabei die Hauptmenge des Diisocyanats bei einer Manteltemperatur von 130—140°C bei einem Durchlaß von 4—6 kg/h abdestilliert; im zweiten Durchgang wird dann

der Rest des Diisocyanats bei gleicher Manteltemperature aber geringerem Durchsatz von etwa 3—4 kg/h entfernt. Man erhält 2058 g eines modifizierten Polyisocyanats mit folgenden Eigenschaften:

| | |
|---|---|
| NCO-Gehalt: | 17,6% |
| Viskosität bei 23°: | 13900 mPas |
| APHA-Farbzahl (DIN 53 409): | 20 |
| Gehalt an monomeren Diisocyanat: | 0,03% |

Das IR-Spektrum zeigt die Anwesenheit von Biuretgruppen (Bande bei 1700 $cm^{-1}$), Urethangruppen (Bande bei 1725 $cm^{-1}$), Uretdiongruppen (schwache Schulter bei 1775 $cm^{-1}$) und Anhydridgruppen (Bande bei 1820 $cm^{-1}$).

Beispiel 2

4032 g (24 Mol) 1,6-Diisocyanatohexan werden in einer Rührapparatur entsprechend Beispiel 1 bei 120°C vorgelegt. In das Diisocyanat trägt man im Verlauf von 1 h 268 g (2 Mol) Dimethylolpropionsäure in fester Form ein. Nach beendeter Zugabe wird noch 1 h bei 120°C gerührt. Insgesamt werden bei der Reaktion korr. 28 l $CO_2$ freigesetzt. Nach Dünnschichtverdampfung des überschüssigen Diisocyanats erhält man 1476 g eines modifizierten Polyisocyanats mit folgenden Eigenschaften:

| | |
|---|---|
| NCO-Gehalt: | 18,4% |
| Viskosität bei 23°C: | 6300 mPas |
| APHA-Farbzahl (DIN 54 409): | 50 |
| Gehalt an monomerem Diisocyanat: | 0,03% |

Beispiel 3

In einer Apparatur entsprechend Beispiel 1 werden 4200 g (25 Mol) 1,6-Diisocyanatohexan und 300 g Phosphorsäuretrimethylester vorgelegt und auf 100°C erhitzt. Dazu tropft man im Verlauf von 2 Stunden eine Lösung von 59 g (0,5 Mol) Hydroxypivalinsäure in 27 g (1,5 Mol) Wasser. Das Säure-Wasser-Gemisch wird dazu auf einer Temperatur von mindestens 50°C gehalten, um es in flüssiger Form dosieren zu können. Nach beendeter Zugabe wird noch 1 h bei 120°C nachgerührt, wobei das Endvolumen des freigesetzten $CO_2$ von korr. 32,9 l erreicht wird. Nach beendeter Reaktion wird das überschüssige Diisocyanat zusammen mit dem Phosphorsäuretrimethylester durch Dünnschichtdestillation entfernt. (Dieses Destillat kann nach Auffüllen mit frischem 1,6-Diisocyanatohexan ohne weitere Reinigungsschritte für Folgepartien eingesetzt werden).

Als Sumpfprodukt erhält man 860 g eines modifizierten Polyisocyanats mit folgenden Eigenschaften:

| | |
|---|---|
| NCO-Gehalt: | 22,8% |
| Viskosität bei 23°: | 5600 mPas |
| APHA-Farbzahl: | 20 |
| Gehalt an monomerem Diisocyanat: | <0,07% |

Beispiel 4

Analog zu Beispiel 3 werden 4536 g (27 Mol) 1,6-Diisocyanatohexan und 600 ml Dioxan mit einem Gemisch aus 80,4 g (0,6 Mol) Dimethylolpropionsäure und 70,2 g (3,0 Mol) Wasser umgesetzt. Im Gegensatz zu Beispiel 3 wird die überwiegende Menge des leichtsiedenden Lösungsmittels vor der Dünnschichtdestillation durch einfache Destillation abgetrennt. Man erhält 1590 g eines modifizierten Polyisocyanats mit den folgenden Eigenschaften:

| | |
|---|---|
| NCO-Gehalt: | 21,6% |
| Viskosität bei 23°C: | 9800 mPas |
| APHA-Farbzahl: | 30 |
| Gehalt an monomerem Diisocyanat: | <0,03% |

Beispiel 5

Analog zu Beispiel 3 werden 4200 g (25 Mol) 1,6-Diisocyanatohexan und 350 g Phosphorsäuretrimethylester mit einer Lösung aus 36 g (2 Mol) Wasser und 11,8 g (0,1 Mol) Hydroxypivalinsäure umgesetzt. Man erhält 906 g eines modifizierten Polyisocyanats mit folgenden Eigenschaften:

| | |
|---|---|
| NCO-Gehalt: | 22,9% |
| Viskosität bei 23°C: | 1580 mPas |
| APHA-Farbzahl: | 30 |
| Gehalt an monomerem Diisocyanat: | <0,03% |

Beispiel 6

Analog zu Beispiel 3 werden 4200 g (20 Mol) 1,6-Diisocyanato-trimethylhexan (technisches Isomerengemisch der 2,2,4- und 2,4,4-trimethylsubstituierten Verbindungen) und 500 ml 1,2-Dimethoxyethan mit 27 g (1,5 Mol) Wasser und 17,4 g (0,1 Mol) 2-Ethyl-2-hydroxymethyl-hexansäure umgesetzt, wobei Wasser und die Säure getrennt, aber synchron dem vorgelegten Diisocyanat zudosiert werden. Man erhält 895 g eines modifizierten Diisocyanats mit folgenden Eigenschaften:

| | |
|---|---|
| NCO-Gehalt: | 18,2% |
| Viskosität bei 23°: | 13600 mPas |
| APHA-Farbzahl: | 20 |
| Gehalt an monomerem Diisocyanat: | <0,07% |

**Patentansprüche**

1. Verfahren zur Herstellung von modifizierten, zugleich Urethan-, Carbonsäureanhydrid- und Biuretgruppen aufweisenden organischen Polyisocyanaten durch Umsetzung von überschüssigen Mengen an organischen Diisocyanaten mit aliphatisch gebundenen Isocyanatgruppen des

Molekulargewichtsbereichs 140—300 mit einem Modifizierungsmittel innerhalb des Temperaturbereichs von 20 bis 160°C, gegebenenfalls in Anwesenheit von mit Wasser mischbaren Lösungsmitteln, und gegebenenfalls unter anschließender Entfernung von überschüssigem, nicht umgesetztem Ausgangsdiisocyanat und sonstigen, gegebenenfalls vorliegenden flüchtigen Bestandteilen aus dem Reaktionsgemisch durch Destillation und/oder Extraktion, dadurch gekennzeichnet, daß man als Modifizierungsmittel

a) Hydroxycarbonsäuren der allgemeinen Formel

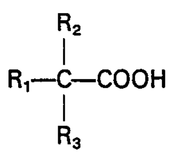

in welcher

$R_1$ einen gegebenenfalls verzweigten, eine primäre oder sekundäre Hydroxylgruppe aufweisenden Alkylrest mit 1 bis 8 Kohlenstoffatomen,

$R_2$ einen gegebenenfalls verzweigten, gegebenenfalls eine primäre oder sekundäre Hydroxylgruppe aufweisenden Alkylrest mit 1 bis 8 Kohlenstoffatomen und

$R_3$ einen gegebenenfalls verzweigten, unsubstituierten Alkylrest mit 1 bis 14 Kohlenstoffatomen

bedeuten, wobei die Summe der Kohlenstoffatome der Reste $R^1$, $R^2$ und $R^3$ 3 bis 20 beträgt und, gegebenenfalls,

b) Wasser

verwendet, wobei ein Molverhältnis von a:b von 0,02:1 bis 1:0 eingehalten wird, und wobei die Menge des Modifizierungsmittels a) oder die Gesamtmenge der Modifizierungsmittelkomponenten a) und b) so bemessen werden, daß das Verhältnis (Mole Ausgangsdiisocyanat):(Gesamtmenge der Mole an Modifizierungsmittelkomponenten a) und b)) bei 3:1 bis 24:1 liegt.

2. Verfahren gemäß Anspruch 1, dadurch gekennzeichnet, daß man als Modifizierungsmittel Monohydroxypivalinsäure oder 2,2-Dimethylolpropionsäure, gegebenenfalls in Form einer wäßrigen Lösung, verwendet.

3. Verfahren gemäß Anspruch 1 und 2, dadurch gekennzeichnet, daß man als Ausgangsdiisocyanat 1,6-Diisocyanatohexan verwendet.

4. Verfahren gemäß Anspruch 1 bis 3, dadurch gekennzeichnet, daß man die Umsetzung innerhalb des Temperaturbereichs von 50 bis 140°C durchführt.

5. Verfahren gemäß Anspruch 1 bis 4, dadurch gekennzeichnet, daß man die Modifizierungsmittelkomponenten a) und b) in einem Molverhältnis a:b von 0,05:1 bis 1:0 verwendet und die Gesamtmenge an Modifizierungsmittel so bemißt, daß das molare Verhältnis Ausgangsdiisocyanat:(Gesamtmenge des Modifizierungsmittels) bei 5:1 bis 15:1 liegt.

6. Verwendung der gemäß Anspruch 1 bis 5 erhältlichen, modifizierten Polyisocyanate, gegebenenfalls in mit Blockierungsmittel für Isocyanatgruppen blockierter Form, als Polyisocyanatkomponente in Polyurethanlack-Systemen.

**Revendications**

1. Procédé de préparation de polyisocyanates organiques modifiés contenant à la fois des groupes uréthanne, des groupes anhydride d'acide carboxylique et des groupes biuret par réaction d'un excès de diisocyanates organiques à groupes isocyanate à liaisons aliphatiques, ayant un poids moléculaire dans l'intervalle de 140 à 300, avec un agent modifiant, dans un intervalle de température de 20 à 160°C, éventuellement en présence de solvants miscibles à l'eau, et éventuellement avec élimination subséquente de l'excès de diisocyanate de départ non converti et d'autres constituants volatils éventuels du mélange de réaction par distillation et/ou extraction, caractérisé en ce que l'on utilise en tant qu'agents modifiants

a) des acides hydroxycarboxyliques de formule générale

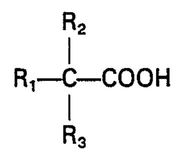

dans laquelle

$R_1$ représente un groupe alkyle en $C_1$—$C_8$ éventuellement ramifié, contenant un groupe hydroxyle primaire ou secondaire,

$R_2$ représente un groupe alkyle en $C_1$—$C_8$ éventuellement ramifié, contenant éventuellement un groupe hydroxyle primaire ou secondaire et

$R_3$ représente un groupe alkyle en $C_1$—$C_4$ éventuellement ramifié, non substitué,

la somme des atomes de carbone des groupes $R_1$, $R_2$ et $R_3$ allant de 3 à 20 et, éventuellement,

b) de l'eau,

en maintenant un rapport molaire a)/b) de 0,02:1 à 1:0, la quantité de l'agent modifiant a) ou la quantité totale des composants a) et b) de l'agent modifiant étant réglée de manière que le rapport (moles du diisocyanate de départ)/(quantité totale des moles des composants a) et b) de l'agent modifiant) aille de 3:1 à 24:1.

2. Procédé selon la revendication 1, caractérisé en ce que l'on utilise en tant qu'agent modifiant l'acide monohydroxypivalique ou l'acide 2,2-diméthylolpropionique, éventuellement à l'état de solution aqueuse.

3. Procédé selon les revendications 1 et 2, caractérisé en ce que l'on utilise en tant que diisocyanate de départ le 1,6-diisocyanatohexane.

4. Procédé selon les revendications 1 à 3, caractérisé en ce que l'on effectue la réaction dans l'intervalle de température de 50 à 140°C.

5. Procédé selon les revendications 1 à 4, caractérisé en ce que l'on utilise les composants a) et b) de l'agent modifiant à un rapport molaire a)/b) de 0,05:1 à 1:0 et on règle la quantité totale d'agent modifiant de manière que le rapport molaire diisocyanate de départ/(quantité totale de l'agent modifiant) aille de 5:1 à 15:1.

6. Utilisation des polyisocyanates modifiés obtenus selon les revendications 1 à 5, éventuellement à l'état bloqué par des agents bloquants pour les groupes isocyanate, en tant que composants polyisocyanates dans des compositions de vernis de polyuréthannes.

**Claims**

1. A process for the production of modified organic polyisocyanates containing urethane, carboxylic anhydride and biuret groups by reaction of excess quantities of organic diisocyanates containing aliphatically bound isocyanate groups and having a molecular weight in the range from 140 to 300 with a modifying agent at temperatures in the range from 20 to 160°C, optionally in the presence of water-miscible solvents and optionally with subsequent removal of excess unreacted starting diisocyanate and any other volatile constituents present from the reaction mixture by distillation and/or extraction, characterized in that the modifying agents used are

a) hydroxycarboxylic acids corresponding to the following general formula

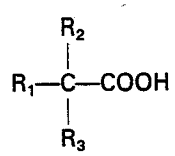

in which

$R_1$ represents an optionally branched $C_1$—$C_8$ alkyl radical containing a primary or secondary hydroxyl group,

$R_2$ represents an optionally branched $C_1$—$C_8$ alkyl radical optionally containing a primary or secondary hydroxyl group and

$R_3$ represents an optionally branched, unsubstituted $C_1$—$C_{14}$ alkyl radical,

the sum of the carbon atoms in the radicals $R^1$, $R^2$ and $R^3$ amounting to between 3 and 20, and, optionally,

b) water

the molar ratio of a to b being from 0.02:1 to 1:0 and the quantity of modifying agent a) or the total quantity of modifying agent components a) and b) being gauged in such a way that the ratio (mols of starting diisocyanate) to (total mols of modifying agent components a) and b)) is from 3:1 to 24:1.

2. A process as claimed in claim 1, characterized in that monohydroxy pivalic acid or 2,2-dimethylol propionic acid, optionally in the form of an aqueous solution, is used as modifying agent.

3. A process as claimed in claims 1 and 2, characterized in that 1,6-diisocyanatohexane is used as the starting diisocyanate.

4. A process as claimed in claims 1 to 3, characterized in that the reaction is carried out at a temperature in the range from 50 to 140°C.

5. A process as claimed in claims 1 to 4, characterized in that the modifying agent components a) and b) are used in a molar ratio of a) to b) of from 0.05:1 to 1:0 and the total quantity of modifying agents is gauged in such a way that the molar ratio of starting diisocyanate to (total quantity of modifying agent) is from 5:1 to 15:1.

6. The use of the modified polyisocyanates obtainable by the process claimed in claims 1 to 5, optionally blocked with blocking agents for isocyanate groups, as polyisocyanate component in polyurethane paint systems.